Europäisches Patentamt

European Patent Office

Office européen des brevets

(11) Veröffentlichungsnummer: **0 103 878**
**B1**

(12) **EUROPÄISCHE PATENTSCHRIFT**

(45) Veröffentlichungstag der Patentschrift: **27.12.90**

(51) Int. Cl.⁵: **A 61 K 31/70, A 61 K 9/08**

(21) Anmeldenummer: **83109239.0**

(22) Anmeldetag: **17.09.83**

(54) **Präparate zur Behandlung von Wunden der Hautoberfläche und Verfahren zur Herstellung derartiger Präparate.**

(30) Priorität: **17.09.82 HU 297482**

(43) Veröffentlichungstag der Anmeldung:
**28.03.84 Patentblatt 84/13**

(45) Bekanntmachung des Hinweises auf die Patenterteilung:
**27.12.90 Patentblatt 90/52**

(84) Benannte Vertragsstaaten:
**AT BE CH DE FR GB IT LI LU NL SE**

(56) Entgegenhaltungen:
EP-A-0 060 553
FR-A-2 160 246

ROTE LISTE, 1961, Seiten 121,122, Editio Cantor, Aulendorff, DE; "Bioget Wund- und Brandtinktur"

(73) Patentinhaber: **Humán Oltoanyagtermelö és Kutato Intézet**
**Táncsics M. u. 82**
**H-2100 Gödöllö (HU)**

(72) Der Erfinder haben auf ihre Nennung verzichtet

(74) Vertreter: **Bartsch, Elisabeth, Dr.**
**Patentanwälte Lotterhos & Partner**
**Lichtensteinstrasse 3**
**D-6000 Frankfurt am Main 1 (DE)**

EP 0 103 878 B1

Courier Press, Leamington Spa, England.

# EP 0 103 878 B1

Es sind zwar schon zahlreiche Verfahren und Präparate zur Behandlung von Verletzungen der Epithelschicht der Haut bekannt, es ist aber auch bekannt, daß das Problem der Behandlung großflächiger Wunden, z.B. das von Brandwunden, immer noch nicht zufriedenstellend gelöst ist.

Brandwunden sind sehr schmerzhaft und rufen einen Schock hervor. Brandwunden ersten Grades sind durch Rötung der Haut bis zur akuten Entzündung, Brandwunden zweiten Grades durch Blasenbildung, verursacht durch ein entzündliches Exsudat, Brandwunden dritten Grades durch Nekrose charakterisiert. Die Schwere der Verbrennungen zweiten und dritten Grades hängt von der Ausdehnung auf der Körperoberfläche ab. Bei der Brandwundenbehandlung muß daher die Hautoberfläche gekühlt, die Wunde gesäubert (Befreiung von Epithelresten, Blasen etc.), der Wundschmerz beseitigt und die Prophylaxe gegen Wundinfektionen eingeleitet werden. Daneben ist die Schockbehandlung durchzuführen.

Die Behandlungen von insbesondere großflächigen Wunden—denen Brandwunden häufig zuzurechnen sind—lassen sich in die sogenannte "offene" und die sogenannte "geschlossene" Behandlung unterteilen. Unter "offener" Behandlungsnmethode ist die Behandlung ohne Verband in einer speziellen keimarmen Umgebung (z.B. unter einem Sauerstoffzelt) zu verstehen, unter "geschlossener" Behandlungsmethode, die Behandlung, bei der die Wunde mit einem Verband gegen Infektionen von außen geschützt wird.

Der "geschlossenen" Method, d.h. der Verbandsmethod wird—wegen der einfacheren Durchführbarkeit—gewöhnlich der Vorzug vor der "offenen" Methode gegeben. Nachteile der Verbandsmethode sind, daß das Verbinden der Wunden sowie das Entfernen des Verbandsmaterials von den Wunden häufig mit Schmerzen verbunden ist. Das Verbandsmaterial kann an den Wunden kleben oder die Wunden nicht vollständig verschließen, was zu Infektionen führen kann.

Eine spezielle Art der "offenen" Behandlung stellt die Anwendung filmbildender Gele dar, die mit dem geronnenen Plasma eine transparente, undurchlässige Schicht auf der Wundoberfläche bilden, mit der zwar die Wunde sicher gegen Infektionen von außen geschützt, mit der aber auch der Abfluß des Exsudats bei Brandwunden zweiten grades verhindert und damit die Bildung von Ödemen begünstigt wird. Bei Brandwunden dritten Grades wird die Gefahr der anaeroben Infektion begünstigt. Bei den für diese Behandlungsmethode verwendeten Gelarten handelt es sich gewöhnlich um Kunststoffe, wie Polyvinylderivate (Vulnoplastin, Aeroplast).

Eine bekannte Behandlungsweise von Wunden ist das "Gerbverfahren", unter Anwendung von Präparaten auf Tannin-Basis (vgl. z.B. FR—A—2 160 246 und "Rote Liste" 1961, S. 121: "Bioget Wund- und Brandtinktur[(R)]). Bei diesem "Gerbverfahren" wird die Wundexsudation herabgesetzt und eine schmerzstillende Wirkung erzielt. Außerdem werden die Proteolyseprodukte gebunden. Diese Methode hat aber den Nachteil, daß ein sehr dicker Schorf entsteht, so daß darunter leicht eine Infektion stattfindet. Die Regeneration unter diesem Schorf ist ungenügend; es kommt oft vor, daß man den Schorf durch eine Operation entfernen muß. Dazu kommt, daß die Behandlung mit Gerbstoffen mit einer etwa 2—5 prozentigen Lösung erfolgt. Bei hohen Konzentrationen können daher die toxischen Nebenwirkungen (Lebernekrose, Kanzerogenität) eine Rolle spielen.

Aus der nicht vorveröffentlichten EP—A—60 553 der Anmelderin sind Präparate zur Behandlung von Wunden der Hautoberfläche sowie Verfahren zur Herstellung dieser Präparate bekannt, die in 100 ml $C_{2-4}$-Alkanol

5—20 mg Gerbstoffe,

5—15 mg Kohlenhydrate,

0,5—6 mg Anthocyane, Verbindungen vom Flavontyp und/oder Pectin,

0,5—6 mg Pflanzenwachse und

0,05—0,1 mg ätherische Öle enthalten.

Die Erfindung betrifft ein Präparat zur Behandlung von Wunden der Hautoberfläche, das in 100 ml $C_{2-4}$-Alkanollösung

bis 20 mg, vorzugsweise 1—20 mg, pflanzliche Gerbstoffe,

bis 50 mg, vorzugsweise 1—50 mg, Zucker,

0,3—6 mg, vorzugsweise 0,5—6 mg, Verbindungen vom Anthocyan- und/oder Flavontyp,

0,1—6 mg, vorzugsweise 0,5—6 mg, Pectin,

bis 6 mg, vorzugsweise 0,1—6 mg, Pflanzenwachs und

0,005—0,1 mg, vorzugsweise 0,01 0,1 mg, ätherische Öle enthält,

| jedoch mit der Ausnahme, daß bei einem | |
|---|---|
| Gerbstoffgehalt | von 5—20 mg, |
| der Gehalt an Zucker | nicht 5—15 mg, |
| an Anthocyan, einer Verbindung vom | |
| Flavontyp und/oder Pectin | nicht 0,5—6 mg |
| und an Pflanzenwachs | |
| betragen darf. | nicht 0,5—6 mg |

Die Präparate der Erfindung können als zusätzliche Komponenten 5—6 mg Vitamine, Spurenelemente,

2

EP 0 103 878 B1

Pflanzenhormone, Enzyme mit oxydativer Wirkung und/oder anorganische Salze enthalten.

Die Erfindung betrifft auch ein Verfahren zur Herstellung von Präparaten zur Behandlung von Wunden der Hautoberfläche, wonach

bis 20 mg, vorzugsweise 1—20 mg, pflanzliche Gerbstoffe,
bis 50 mg, vorzugsweise 1—50 mg, Zucker,
03,—6 mg, vorzugsweise 0,5—6 mg, Verbindungen vom Anthocyan- und/oder Flavontyp,
0,1—6 mg, vorzugsweise 0,5—6 mg, Pectin,
bis 6 mg, vorzugsweise 0,1—6 mg, Pflanzenwachs und
0,005—0,1 mg, vorzugsweise 0,01 0,1 mg, ätherische Öle enthält, auf einmal oder verteilt in $C_{2-4}$-Alkanol zu 100 ml Lösung gelöst werden,

jedoch mit der Ausnahme, daß bei Verwendung

| | |
|---|---|
| einer Gerbstoffmenge | von 5—20 mg, |
| die verwendete Menge an Zucker | nicht 5—15 mg und |
| an Anthocyan, einer Verbindung vom Flavontyp und/oder Pectin | nicht 0,5—6 mg |
| betragen darf. | |

Als Lösungsmittel wird zweckmäßig 70—100 %iges, vorzugsweise 96 %iges, Äthanol verwendet.

Als zusätzliche Komponenten können

5—6 mg Vitamine, Spurenelemente, Pflanzenhormone, Enzyme mit oxydativer Wirkung und/oder anorganische Salze verwendet werden.

Einige Beispiele

für pflanzliche Gerbstoffe sind: Brenzcatechin, Tannin, Gallussäure, Digallussäure sowie Pentadigalloyl-glucose,

für Zucker: Glucose, Fructose, Ribose, Arabinose, Rhamnose sowie Xylose,

für Verbindungen vom Anthocyantyp Anthocyan, Cyanidin, Pelargonidin, Delphinin sowie Derivate dieser Verbindungen,

für Verbindungen vom Flavontyp: Quercetin, Apigenin, Kämpferol sowie Morin,

für ätherische Öle: Geraniol (40—90%), Nerol (4—15%), Citronellol, Eugenol, Farnesol und/oder Linalool bzw. in Form der entsprechenden Aldehyde, wie Citral (10—50%).

Einige Beispiele für die Zusatzkomponenten sind:

für die Vitamine: Vitamin A, $B_1$, $B_2$, $B_6$, $B_{12}$, C, K sowie P,

für die anorganischen Salze: Phosphate sowie Chloride,

für die Spurenelemente: Mangan, Magnesium, Calcium, Eisen, Zink, Kobalt, Kupfer, Molybdän sowie Nickel,

für die Pflanzenhormone: Auxin, Kinetin (in ng-Mengen) und

für die Enzyme mit oxydativer Wirkung: Peroxydase sowie Katalase.

Die einzelnen Komponenten können gemeinsam in Äthanol gelöst werden, wenn es sich um Äthanollösliche Materialien handelt. Es ist zweckmäßig die einzelnen Zusatzstoffe zuerst ineinander oder in wenig Wasser aufzulösen und erst danach in das Präparat umzulösen. Gegebenenfalls kann ein Lösungsvermittler angewendet werden (z.B. Äther).

Es hat sich als zweckmäßig erwiesen, wenn die Vitamine A und K in ätherischen Ölen gelöst dem System zugegeben werden. Das Vitamin C kann in konzentrierter wäßriger Lösung zugesetzt werden.

Das Lösen der Komponenten erfolgt vorzugsweise bei 0—30°C, also bei niedrigerer Temperatur als Raumtemperatur. Aus den einzelnen Komponenten werden zweckmäßig Stammlösungen—möglichst in Äthanol aber auch in Wasser—hergestellt, mit denen dann bei der Herstellung der Endkomposition die genaue Dosierung vorgenommen wird. Die Stammlösungen sollen bis zum Verbrauch in einem dunklen, kühlen Raum aufbewahrt werden.

Die Äthanollösung, die die Hauptkomponenten enthält, ist sehr stabil und kann lange Zeit bei Raumtemperatur gelagert werden, sie ist aber vor Sonnenlicht zu schützen. Lösungen, deren Gerbstoff- und Wachsgehalt in die niedrigen Mengenbereiche fällt, wiesen—bei höheren Zucker-Konzentrationen—eine erhöhte Stabilität auf, die über zwei Jahre beträgt.

Die einzelnen Komponenten können unmittelbar als solche oder als Pflanzenextrakte dem Präparat zugegeben werden.

Zur Anwendung wird das Präparat der Erfindung auf die gereinigte Wundoberfläche (bei Verätzungen z.B. nach Waschen und Neutralisieren der Wunden)—vorzugsweise durch Aufsprühen—in dünner Schicht aufgebracht. Diese Behandlung soll so oft wiederholt werden, bis das Schmerzgefühl nachläßt. Die Wundoberfläche soll während der Behandlung vor Wasser und Seife geschützt werden.

Aus dem auf die Wundoberfläche aufgesprühten Präparat der Erfindung verdunstet der Alkohol, womit ein Kühleffekt auf der Wunde erzielt wird,—was bei der Anwendung des Präparates als erste Hilfe wichtig ist—und es bildet sich auf der Wundoberfläche eine dünne Filmschicht, die winzige Atmungsöffnungen (Vakuolen) enthält. Der Durchmesser der Atmungsöffnungen beträgt maximal 0,2 µm und ist damit für Bakterien undurchgängig. Die Bildung dieser Filmschicht wird auf die Anwesenheit der Pflanzenwachse und der Pectine zurückgeführt.

3

Die in dem Präparat der Erfindung und dann in der Filmschicht enthaltenen pflanzlichen Gerbstoffe üben eine adstringierende, bakterizide und viruzide Wirkung aus; außerdem fördern sie die Schorfbildung. Durch Anwendung von erhöhten Konzentrationen an Gerbstoffen werden toxische Nebenwirkungen vermieden.

Die Verbindungen vom Anthocyan- und vom Flavontyp weisen in der Kombination der Erfindung eine bakteriostatische Wirkung auf; außerdem üben sie eine Wirkung auf die Kapillargefäße aus und setzen die Mikrozirkulation in den verletzten Bereichen in Gang, wodurch eine schmerzstillende Wirkung erzielt und die interstitiell gebildeten Ödeme abgebaut werden.

Die Zucker, die teilweise mit den Anthocyanen in Glykosidbildung, teilweise in freier Form vorliegen, wirken auf die Anthocyane bzw. Flavone aktivierend und unterstützen damit deren Wirkung.

Die ätherischen Öle sind bakteriostatisch wirksam, und die Enzyme setzen den Sauerstoff aus den in den Gewebszellen vorhandenen Peroxyden frei. Sie greifen in die Oxidationsprozesse der Anthocyane und/oder Flavone ein und fördern damit deren Wirkung auf die Wundheilung.

Die Spurenelemente begünstigen die Oxydations-Reduktionsprozesse; sie unterstützen damit gemeinsam mit den Pflanzenhormonen und den Enzymen die Regeneration der Haut.

Zusammenfassend ist festzustellen, die bei Anwendung des Präparates der Erfindung beobachtete bakteriostatische Wirkung ist auf die Kombinierte Wirkung von dem als Lösungsmittel benutzten Alkohol, den pflanzlichen Gerbstoffen, den Verbindungen vom Anthocyan- und Flavontyp und den ätherischen Ölen zurückzuführen, die Regeneration der Haut dagegen auf die Anwesenheit der Pflanzenhormone, der Enzyme und der Spurenelemente. Die beobachtete analgetische Wirkung ist teilweise auf die Bildung einer atmungsaktiven Filmschicht, die den Abfluß von Wundsekret (Exsudat) zuläßt, aber den Zutritt von Bakterien verhindert, auf der Wundoberfläche und teilweise darauf zurückzuführen, daß mit der unter der Filmschicht einsetzenden Mikrozirkulation interstitielle Ödeme abgebaut werden.

Die Präparate der Erfindung können nicht nur zur Behandlung von oberflächlichen oder tieferen Brandwunden, sondern auch zur Behandlung von Beingeschwüren verschiedenen Ursprungs, chirurgischen Wunden, eiternden Wunden, geöffneten Abszessen, von Allergo-Dermatosen, die auf Kokkeninfektionen zurückgehen, Pyodermien, von Hautausschlägen, die durch Herpes-Virus verursacht sind, und bei plastischen Operationen zur Behandlung des Transplantats sowie des Bereichs, aus dem die Hautstücke entnommen sind, verwendet werden.

Die Erfindung soll durch folgende Beispiele erläutert werden:

Beispiel 1

Mit 96 %igem Äthanol wird eine Lösung aus folgenden Ingredientien hergestellt:

2 g Gallusgerbsäure,
0,4 g Cyanin,
0,05 g Apigenin,
0,05 g Delphinin,
0,2 g Quercetin,
15 g Glucose,
9 g Fructose,
2 g Xylose,
1 g Rhamnose,
1 g Ribose,
2,8 g Pectin,
0,1 g Bienenwachs,
0,1 g Carnaubawachs,
0,0045 g Geraniol,
0,0015 g Nerol,
0,003 g Citronellol,
0,003 g Citral,
0,005 g Peroxydase-Katalase,
0,00014 g Vitamin $B_1$,
0,00007 g Vitamin $B_2$,
0,00006 g Vitamin $B_6$,
0,000001 g Vitamin $B_{12}$,
0,00002 g Vitamin C,
0,0008 g Calcium-pantothenat,
0,0013 g Nikotinsäure,
0,05 g Eisen(III)chlorid,
0,03 g Mangan(II)chlorid,
0,02 g Cobalt(II)chlorid,
0,02 g Magnesiumchlorid,
0,05 g Dinatrium-hydrogen-phosphat und
0,5 g Kalium-dihydrogen-phosphat.

Die erhaltene Lösung wird zunächst mit Äthanol auf 1,000 ml und dann mit 96 %igem Äthanol auf 100 l verdünnt, steril filtriert und abgepackt.

Beispiel 2

in 100 ml 96 %igem Äthanol werden wie in Beispiel 1

0,4 g Gallusgerbsäure,
1,0 g Pentadigalloyl-glucose,
0,1 g Brenzcatechingerbsäure,
0,5 g Fructose,
1,8 g Glucose,
0,2 g Rhamnose,
0,15 g Cyanin,
0,05 g Apigenin,
0,2 g Bienenwachs,
0,2 g Carnaubawachs,
0,006 g Geraniol,
0,001 g Nerol,
0,001 g Citronellol,
0,00011 g Vitamin $B_1$,
0,00004 g Vitamin C,
0,00005 g Nikotinsäure,
0,005 g Peroxydase-Katalase und
0,001 g Kinetin, 0,5 g Pectin

gelöst und das erhaltene Konzentrat auf das 100-fache verdünnt.

Beispiel 3

In 100 ml 96 %igem Äthanol werden wie in Beispiel 1

0,22 g Gallusgerbsäure,
1,0 g Glucose,
0,4 g Fructose,
0,3 g Rhamnose,
0,2 g Xylose,
0,1 g Ribose,
0,04 g Cyanin,
0,00015 g Vitamin $B_1$,
0,00003 g Vitamin C,
0,00007 g Nikotinsäure,
0,005 g Peroxydase-Katalase,
0,001 g Kinetin,
0,005 g Apigenin,
0,005 g Delphinin,
0,01 g Quercetin,
0,01 g Kämpferol,
0,35 g Pectin,
0,2 g Bienenwachs,
0,2 g Carnaubawachs,
0,005 g Geraniol,
0,001 g Nerol,
0,001 g Citronellol und
0,001 g Citral

gelöst und das erhaltene Konzentrat auf das 100-fache verdünnt.

Beispiel 4

In 100 ml 96 %igem Äthanol werden wie in Beispiel 1

0,4 g Gallusgerbsäure,
0,1 g Brenzcatechingerbsäure,
1,0 g Pentadigalloyl-glucose,
2,0 g Glucose,
0,5 g Fructose,
0,3 g Rhamnose,
0,2 g Xylose,
0,04 g Cyanin,
0,005 g Apigenin,

EP 0 103 878 B1

0,005 g Delphinin,
0,01 g Quercetin,
0,00007 g Nikotinsäure,
0,005 g Peroxydase-Katalase,
0,001 g Kinetin,
0,01 g Kämpferol,
0,28 g Pectin,
0,2 g Bienenwachs,
0,2 g Carnaubawachs,
0,005 g Geraniol,
0,001 g Nerol,
0,001 g Citronellol,
0,001 g Citral,
0,00015 g Vitamin $B_1$ und
0,00003 g Vitamin C.

gelöst und das erhaltene Konzentrat auf das 100-fache verdünnt.

Parenterale toxikologische Untersuchungen

Die Untersuchungen der akuten Toxizität wurden nach den Regeln für das Eintragen eines Arzneimittels ins Arzneibuch durchgeführt.

Das Mittel ist zur lokalen Behandlung von verbrannten Hautflächen, bzw. von Hautflächen ohne Epithelschicht geeignet. Im Laufe der pharmakologischen präklinischen Untersuchungen wurden mit dem Mittel 120 Kaninchen, 40 Ratten und 50 Meerschweinchen mit Brandwunden 3 bis 4 Wochen behandelt. Innerhalb dieser Zeit konnte an den Tieren keine auf toxische Wirkung hinweisende krankhafte Veränderung wahrgenommen werden, was als Grundlage der Untersuchung der lokalen Toxizität gelten kann. Obwohl das Arzneimittel nicht absorbiert wird und ausschließlich zur lokalen Behandlung dient, wurden auch die parenteralen Untersuchungen vorgenommen. Da bei der parenteralen Verabreichung mit der Wirkung des Alkohols gerechnet werden muß, wurde für die Untersuchungen das Mittel eingedampft und der Rückstand mit 1 %iger $NaHCO_3$-Lösung bei pH 8,2 erneut gelöst und die Lösung sterilfiltriert.

Die Dosis wurde auf Grund des Gehaltes an Trockensubstanz bestimmt, die maximale Dosis war bei Mäusen 0,5 ml, bei Kaninchen 5,0 ml.

Bei den zu untersuchenden Dosen war der Ausgangspunkt die Menge, die in der Humantherapie an einem Tag maximal auf die Haut des Patienten gelangen kann. Bei Annahme eines Durchschnittsgewichtes von 60 kg und 500 ml Mittel ergibt das 3,0 mg/kg als Tagesdosis, die ausschließlich auf die Hautoberfläche gelangt.

Parenterale Untersuchung der akuten Toxizität

| Tier | Anzahl | Appl. | mg/kg | wievielfaches der Humandosis | Beobach- tungszeit | Ergebnis |
|------|--------|-------|-------|------------------------------|---------------------|----------|
| Maus | 12 | i.p. | 38,5 | 13× | 2 Monate | Ø |
| Maus | 12 | i.p. | 77,0 | 26× | 2 Monate | 2 am 23. Tag |
| Kaninchen | 12 | i.v. | 8,0 | 2,7× | 2 Wochen bis 2 Monate | Ø |
| Kaninchen | 5 | i.v. | 20,0 | 6,7× | „ | Ø |
| Kaninchen | 5 | i.v. | 100,0 | 33× | „ | Ø |

Aus der Tabelle ist ersichtlich, daß das Mehrfache der maximal auf die menschliche Haut gelangenden Dosis weder für Mäuse noch für Kaninchen toxisch ist.

Anschließend wurde die $LD_{50}$ bestimmt. Dazu wurden Mäusen des Stammes CLFP LATI i.v. mit dem eingedampften, in $NaHCO_3$-Lösung wieder aufgelösten Mittel geimpft, jede Gruppe umfaßte 20 Tiere. Bei statistischer Auswertung der erhaltenen Ergebnisse wurde gefunden, daß der $LD_{50}$-Wert 196,0±36 mg/kg beträgt, d.h. das 50fache der ganztägigen menschlichen Dosis.

Behandlung

Die Lösung gemäß Beispiel 1 (nachfolgend: "Mittel") wurde zur Behandlung von 98 mit

6

Verbrennungswunden ins Krankenhaus eingelieferten Kindern angewendet, 25% der Kranken hatten Verbrennungen dritten Grades.

Behandlungsweise:

Das Mittel wurde aus etwa 20 cm Entfernung direkt auf die verletzte Fläche gesprüht, und zwar reichlich, bis zum Abtropfen. Wo dazu die Möglichkeit bestand, wurde-statt des als Erste Hilfe empfohlenen Kühlens mit kaltem Wasser-das Mittel auch zum Wärmeentzug verwendet. Bei Brandwunden sehr großer Ausdehnung wurden vor der Behandlung schmerzstillende Mittel verabreicht. Vor der Behandlung wurden vorhandene Blasen entfernt, Salben- und sonstige Reste ebenso.

Die erste Behandlung wurde so lange fortgesetzt, bis der Patient schmerzfrei war (innerhalb von 30 bis 40 Minuten 6 bis 8 Sprühbehandlungen). Später wurde täglich 3 bis 4 mal bzw. täglich einmal behandelt. Während der ganzen Zeit kamen die kranken Flächen mit Wasser oder wäßrigen Lösungen nicht in Berührung.

Verglichen mit den bekannten Methoden der Behandlung (z.B. Sulfodiazin-Silbernitrat-Salbe, Mercurochromlösung) weist die erfindungsgemäße Behandlung folgende Vorteile auf:

1. Die durchschnittliche Dauer des Krankenhausaufenthaltes sank um 28%.

2. Bei einem großen Teil der Kranken konnte nach einigen Tagen die Behandlung als ambulante Behandlung fortgesetzt werden.

3. Wurde das Mittel sofort eingesetzt, so waren im allgemeinen keine plastischen Operationen erforderlich.

4. Wurde das Mittel bereits bei der Ersten Hilfe eingesetzt, so war die Kompensierung des Brandschocks leichter. Nach 24 bis 36 Stunden bildete sich Grind, der den Verlust an Plasma bzw. Feuchtigkeit sowie Infektionen verhinderte.

5. 25 bis 30% weniger Humanplasma, Flüssigkeit und kalorischer Ersatz waren erforderlich als bei anderen Verfahren.

6. Wird sofort mit dem Mittel behandelt, so ist weder parenterale noch lokale Behandlung mit Antibiotika erforderlich. Wurde zuerst mit etwas anderem behandelt, so genügte nach der Umstellung auf das Mittel eine lokale Behandlung mit Antibiotikum.

7. Brandsepsis, Leber- oder Nierenschäden traten auch bei den Fällen dritten Grades nicht auf.

8. Die Untersuchung der lebenswichtigen Organe (Nieren, Leber) und des Bluts zeigten, daß weder eine giftige noch eine allergische Nebenwirkung vorliegt.

9. Bei offener Behandlung (ohne Verband) trat die Bildung von Grind immer ein, Unter dem Grind sammelte sich niemals Flüssigkeit an.

10. Die Behandlung selbst verursachte nur in den ersten 24 Stunden 1 bis 2 Minuten Schmerzen. Nach der Bildung des Schorfs ist die Behandlung schmerzlos und viele Kranke (größere Kinder) konnten sich selbst behandeln.

11. Im Vergleich mit den bekannten Behandlungsmethoden sind die Krankenhauskosten etwa 40% geringer.

**Beschreibung für die Vertragsstaaten: LU SE**

Es sind zwar schon zahlreiche Verfahren und Präparate zur Behandlung von Verletzungen der Epithelschicht der Haut bekannt, es ist aber auch bekannt, daß das Problem der Behandlung großflächiger Wunden, z.B. das von Brandwunden, immer noch nicht zufriedenstellende gelöst ist.

Brandwunden sind sehr schmerzhaft und rufen einen Schock hervor. Brandwunden ersten Grades sind durch Rötung der Haut bis zur akuten Entzündung, Brandwunden zweiten Grades durch Blasenbildung, verursacht durch ein entzündliches Exsudat, Brandwunden dritten Grades durch Nekrose charakterisiert. Die Schwere der Verbrennungen zweiten und dritten Grades hängt von der Ausdehnung auf der Körperoberfläche ab. Bei der Brandwundenbehandlung muß daher die Hautoberfläche gekühlt, die Wunde gesäubert (Befreiung von Epithelresten, Blasen etc.), der Wundschmerz beseitigt und die Prophylaxe gegen Wundinfektionen eingeleitet werden. Daneben ist die Schockbehandlung durchzuführen.

Die Behandlungen von insbesondere großflächigen Wunden—denen Brandwunden häufig zuzurechnen sind—lasse sich in die sogenannte "offene" und die sogenannte "geschlossene" Behandlung unterteilen. Unter "offener" Behandlungsmethode ist die Behandlung ohne Verband in einer speziellen keimarmen Umgebung (z.B. unter einem Sauerstoffzelt) zu verstehen, unter "geschlossener" Behandlungsmethode, die Behandlung, bei der die Wunde mit einem Verband gegen Infektionen von außen geschützt wird.

Der "geschlossenen" Methode, d.h. der Verbandsmethode wird—wegen der einfacheren Durchführbarkeit—gewöhnlich der Vorzug vor der "offenen" Methode gegeben. Nachteile der Verbandsmethode sind, daß das Verbinden der Wunden sowie das Entfernen des Verbandsmaterials von den Wunden häufig mit Schmerzen verbunden ist. Das Verbandsmaterial kann an den Wunden kleben oder die Wunden nicht vollständig verschließen, was zu Infektionen führen kann.

Eine spezielle Art der "offenen" Behandlung stellt die Anwendung filmbildender Gele dar, die mit dem geronnen Plasma eine transparente, undurchlässige Schicht auf der Wundoberfläche bilden, mit der zwar die Wunde sicher gegen Infektionen von außen geschützt, mit der aber auch der Abfluß des Exsudats bei Brandwunden zweiten Grades verhindert und damit die Bildung von Ödemen begünstigt wird. Bei Brandwunden dritten Grades wird die Gefahr der anaeroben Infektion begünstigt. Bei den für diese Behandlungsmethode verwendete Gelarten handelt es sich gewöhnlich um Kunststoffe, wie Polyvinylderivate (Vulnoplastin, Aeroplast).

Eine bekannte Behandlungsweise von Wunden ist das "Gerbverfahren", unter Anwendung von Präparaten auf Tannin-Basis (vgl. z.B. FR—A—2 160 246 und "Rote Liste" 1961, S. 121: "Bioget Wund- und Brandtinktur"[(R)]). Bei diesem "Gerbverfahren" wird die Wundexsudation herabgesetzt und eine schmerzstillende Wirkung erzielt. Außerdem werden die Proteolyseprodukte gebunden. Diese Methode hat aber den Nachteil, daß ein sehr dicker Schorf entsteht, so daß darunter leicht eine Infektion stattfindet. Die Regeneration unter diesem Schorf ist ungenügend; est kommt oft vor, daß man den Schorf durch eine Operation entfernen muß. Dazu kommt, daß die Behandlung mit Gerbstoffen mit einer etwa 2—5 prozentigen Lösung erfolgt. Bei hohen Konzentrationen können daher die toxischen Nebenwirkungen (Lebernekrose, Kanzerogenität) eine Rolle spielen.

Die Erfindung betrifft ein Präparat zur Behandlung von Wunden der Hautoberfläcge, das in 100 ml $C_{2-4}$-Alkanollösung

bis 20 mg, vorzugsweise 1—20 mg, pflanzliche Gerbstoffe,

bis 50 mg, vorzugsweise 1—50 mg, Zucker,

0,3—6 mg, vorzugsweise 0,5—6 mg, Verbindungen vom Anthocyan- und/oder Flavontyp,

0,1—6 mg, vorzugsweise 0,5—6 mg, Pectin,

bis 6 mg, vorzugsweise 0,1—6 mg, Pflanzenwachs und

0,005—0,1 mg, vorzugsweise 0,01 0,1 mg, ätherische Öle enthält,

Die Präparate der Erfindung können als zusätzliche Komponenten 5—6 mg Vitamine, Spurenelemente, Pflanzenhormone, Enzyme mit oxydativer Wirkung und/oder anorganische Salze enthalten.

Die Erfindung betrifft auch ein Verfahren zur Herstellung von Präparaten zur Behandlung von Wunden der Hautoberfläche, wonach

bis 20 mg, vorzugsweise 1—20 mg, pflanzliche Gerbstoffe,

bis 50 mg, vorzugsweise 1—50 mg, Zucker,

0,3—6 mg, vorzugsweise 0,5—6 mg, Verbindungen vom Anthocyan- und/oder Flavontyp,

0,1—6 mg, vorzugsweise 0,5—6 mg, Pectin,

bis 6 mg, vorzugsweise 0,1—6 mg, Pflanzenwachs und

0,005—0,1 mg, vorzugsweise 0,01 0,1 mg, ätherische Öle enthält,

auf einmal oder verteilt in $C_{2-4}$-Alkanol zu 100 ml Lösung gelöst werden.

Als Lösungsmittel wird zweckmäßig 70—100 %iges, vorzugsweise 96 %iges, Äthanol verwendet.

Als zusätzliche Komponenten können

5—6 mg Vitamine, Spurenelemente, Pflanzenhormone, Enzyme mit oxydativer Wirkung und/oder anorganische Salze verwendet werden.

Einige Beispiele

für pflanzliche Gerbstoffe sind: Brenzcatechin, Tannin, Gallussäure, Digallussäure sowie Pentadigalloyl-glucose,

für Zucker: Glucose, Fructose, Ribose, Arabinose, Rhamnose sowie Xylose,

für Verbindungen vom Anthocyantyp Anthocyan, Cyanidin, Pelargonidin, Delphinin sowie Derivate dieser Verbindungen,

für Verbindungen vom Flavontyp: Quercetin, Apigenin, Kämpferol sowie Morin,

für ätherische Öle: Geraniol (40—90%), Nerol (4—15%), Citronellol, Eugenol, Farnesol und/oder Linalool bzw. in Form der entsprechenden Aldehyde, wie Citral (10—50%).

Einige Beispiele für die Zusatzkomponenten sind:

für die Vitamine: Vitamin A, $B_1$, $B_2$, $B_6$, $B_{12}$, C, K sowie P,

für die anorganischen Salze: Phosphate sowie Chloride,

für die Spurenelemente: Mangan, Magnesium, Calcium, Eisen, Zink, Kobalt, Kupfer, Molybdan sowie Nickel,

für die Pflanzenhormone: Auxin, Kinetin (in ng-Mengen) und

für die Enzyme mit oxydativer Wirkung: Peroxydase sowie Katalase.

Die einzelnen Komponenten können gemeinsam in Äthanol gelöst werden, wenn es sich um Äthanollösliche Materialien handelt. Es ist zweckmäßig die einzelnen Zusatzstoffe zuerst ineinander oder in wenig Wasser aufzulösen und erst danach in das Präparat umzulösen. Gegebenenfalls kann ein Lösungsvermittler angewendet werden (z.B. Äther).

Es hat sich als zweckmäßig erwiesen, wenn die Vitamine A und K in ätherischen Ölen gelöst dem System zugegeben werden. Das Vitamin C kann in konzentrierter wäßriger Lösung zugesetzt werden.

Das Lösen der Komponenten erfolgt vorzugsweise bei 0—30°C, also bei niedriger Temperatur als

8

Raumtemperatur. Aus den einzelnen Komponenten werden zweckmäßig Stammlösungen—möglichst in Äthanol aber auch in Wasser—hergestellt, mit denen dann bei der Herstellung der Endkomposition die genaue Dosierung vorgenommen wird. Die Stammlösungen sollen bis zum Verbrauch in einem dunklen, kühlen Raum aufbewahrt werden.

Die Äthanollösung, die die Hauptkomponenten enthält, ist sehr stabil und kann lange Zeit bei Raumtemperatur gelagert werden, sie ist aber vor Sonnenlicht zu schützen. Lösungen, deren Gerbstoff- und Wachsgehalt in die niedrigen Mengenbereiche fällt, wiesen—bei höheren Zucker-Konzentrationen—eine erhöhte Stabilität auf, die über zwei Jahre beträgt.

Die einzelnen Komponenten können unmittelbar als solche oder als Pflanzenextrakte dem Präparat zugegeben werden.

Zur Anwendung wird das Präparat der Erfindung auf die gereinigte Wundoberfläche (bei Verätzungen z.B. nach Waschen und Neutralisieren der Wunden)—vorzugsweise durch Aufsprühen—in dünner Schicht aufgebracht. Diese Behandlung soll so oft wiederholt werden, bis das Schmerzgefühlt nachläßt. Die Wundoberfläche soll während der Behandlung vor Wasser und Seife geschützt werden.

Aus dem auf die Wundoberfläche aufgesprühten Präparat der Erfindung verdunstet der Alkohol, womit ein Kühleffekt auf der Wunde erzielt wird,—was bei der Anwendung des Präparates als erste Hilfe wichtig ist—und es bildet sich auf der Wundoberfläche eine dünne Filmschicht, die winzige Atmungsöffnungen (Vakuolen) enthält. Der Durchmesser der Atmungsöffnungen beträgt maximal 0,2 μm und ist damit für Bakterien undurchgängig. Die Bildung dieser Filmschicht wird auf die Anwesenheit der Pflanzenwachse und der Pectine zurückgeführt.

Die in dem Präparat der Erfindung und dann in der Filmschicht enthaltenen pflanzlichen Gerbstoffe üben eine adstringierende, bakterizide und viruzide Wirkung aus; außerdem fördern sie die Schorfbildung. Durch Anwendung von erhöhten Konzentrationen an Gerbstoffen werden toxische Nebenwirkungen vermieden.

Die Verbindungen vom Anthocyan- und vom Flavontyp weisen in der Kombination der Erfindung eine bakteriostatische Wirkung auf; außerdem üben sie eine Wirkung auf die Kapillargefäße aus und setzen die Mikrozirkulation in den verletzten Bereichen in Gang, wodurch eine schmerzstillende Wirkung erzielt und die interstitielle gebildeten Ödeme abgebaut werden.

Die Zucker, die teilweise mit den Anthocyanen in Glykosidbildung, teilweise in freier Form vorliegen, wirken auf die Anthocyane bzw. Flavone aktivierend und unterstützen damit deren Wirkung.

Die ätherischen Öle sind bakteriostatisch wirksam, und die Enzyme setzen den Sauerstoff aus den in den Gewebszellen vorhandenen Peroxyden frei. Sie greifen in die Oxydationsprozesse der Anthocyane und/oder Flavone ein und fördern damit deren Wirkung auf die Wundheilung.

Die Spurenelemente begünstigen die Oxydations-Reduktionsprozesse; sie unterstützen damit gemeinsam mit den Pflanzenhormonen und den Enzymen die Regeneration der Haut.

Zusammenfassend ist festzustellen, die bei Anwendung des Präparates der Erfindung beobachtete bakteriostatische Wirkung ist auf die Kombinierte Wirkung von dem als Lösungsmittel benutzten Alkohol, den pflanzlichen Gerbstoffen, den Verbindungen vom Anthocyan- und Flavontyp und den ätherischen Ölen zurückzuführen, die Regeneration der Haut dagegen auf die Anwesenheit der Pflanzenhormone, der Enzyme und der Spurenelemente. Die beobachtete analgetische Wirkung ist teilweise auf die Bildung einer atmungsaktiven Filmschicht, die den Abfluß von Wundsekret (Exsudat) zuläßt, aber den Zutritt von Bakterien verhindert, auf der Wundoberfläche und teilweise darauf zurückzuführen, daß mit der unter der Filmschicht einsetzenden Mikrozirkulation interstitielle Ödeme abgebaut werden.

Die Präparate der Erfindung können nicht nur zur Behandlung von oberflächlichen oder tieferen Brandwunden, sondern auch zur Behandlung von Beingeschwüren verschiedenen Ursprungs, chirurgischen Wunden, eiternden Wunden, geöffneten Abszessen, von Allergo-Dermatosen, die auf Kokkeninfektionen zurückgehen, Pyodermien, von Hautausschlägen, die durch Herpes-Virus verursacht sind, und bei plastischen Operationen zur Behandlung des Transplantats sowie des Bereichs, aus dem die Hautstücke entnommen sind, verwendet werden.

Die Erfindung soll durch folgende Beispiele erläutert werden.

Beispiel 1

Mit 96 %igem Äthanol wird eine Lösung aus folgenden Ingredientien hergestellt:

2 g Gallusgerbsäure,
0,4 g Cyanin,
0,05 g Apigenin,
0,05 g Delphinin,
0,2 g Quercetin,
15 g Glucose,
9 g Fructose,
2 g Xylose,
1 g Rhamnose,
1 g Ribose,
2,8 g Pectin,
0,1 g Bienenwachs,

0,1 g Carnaubawachs,
0,0045 g Geraniol,
0,0015 g Nerol,
0,003 g Citronellol,
0,003 g Citral,
0,005 g Peroxydase-Katalase,
0,00014 g Vitamin $B_1$,
0,00007 g Vitamin $B_2$,
0,00006 g Vitamin $B_6$,
0,000001 g Vitamin $B_{12}$,
0,00002 g Vitamin C,
0,0008 g Calcium-pantothenat,
0,0013 g Nikotinsäure,
0,05 g Eisen(III)chlorid,
0,03 g Mangan(II)chlorid,
0,02 g Cobalt(II)chlorid,
0,02 g Magnesiumchlorid,
0,05 g Dinatrium-hydrogen-phosphat und
0,5 g Kalium-dihydrogen-phosphat.

Die erhaltene Lösung wird zunächst mit Äthanol auf 1,000 ml und dann mit 96 %igem Äthanol auf 100 l verdünnt, steril filtriert und abgepackt.

Beispiel 2

in 100 ml 96 %igem Äthanol werden wie in Beispiel 1
0,4 g Gallusgerbsäure,
1,0 g Pentadigalloyl-glucose,
0,1 g Brenzcatechingerbsäure,
0,5 g Fructose,
1,8 g Glucose,
0,2 g Rhamnose,
0,15 g Cyanin,
0,05 g Apigenin,
0,2 g Bienenwachs,
0,2 g Carnaubawachs,
0,006 g Geraniol,
0,001 g Nerol,
0,001 g Citronellol,
0,00011 g Vitamin $B_1$,
0,00004 g Vitamin C,
0,00005 g Nikotinsäure,
0,005 g Peroxydase-Katalase und
0,001 g Kinetin, 0,5 g Pectin,

gelöst und das erhaltene Konzentrat auf das 100-fache verdünnt.

Beispiel 3

In 100 ml 96 %igem Äthanol werden wie in Beispiel 1
0,22 g Gallusgerbsäure,
1,0 g Glucose,
0,4 g Fructose,
0,3 g Rhamnose,
0,2 g Xylose,
0,1 g Ribose,
0,04 g Cyanin,
0,00015 g Vitamin $B_1$,
0,00003 g Vitamin C,
0,00007 g Nikotinsäure,
0,005 g Peroxydase-Katalase,
0,001 g Kinetin,
0,005 g Apigenin,
0,005 g Delphinin,
0,01 g Quercetin,
0,01 g Kämpferol,
0,35 g Pectin,

0,2 g Bienenwachs,
0,2 g Carnaubawachs,
0,005 g Geraniol,
0,001 g Nerol,
0,001 g Citronellol und
0,001 g Citral

gelöst und das erhaltene Konzentrat auf das 100-fache verdünnt.

Beispiel 4
In 100 ml 96 %igem Äthanol werden wie in Beispiel 1
0,4 g Gallusgerbsäure,
0,1 g Brenzcatechingerbsäure,
1,0 g Pentadigalloyl-glucose,
2,0 g Glucose,
0,5 g Fructose,
0,3 g Rhamnose,
0,2 g Xylose,
0,04 g Cyanin,
0,005 g Apigenin,
0,005 g Delphinin,
0,01 g Quercetin,
0,00007 g Nikotinsäure,
0,005 g Peroxydase-Katalase,
0,001 g Kinetin,
0,01 g Kämpferol,
0,28 g Pectin,
0,2 g Bienenwachs,
0,2 g Carnaubawachs,
0,005 g Geraniol,
0,001 g Nerol,
0,001 g Citronellol,
0,001 g Citral,
0,00015 g Vitamin $B_1$ und
0,00003 g Vitamin C

gelöst und das erhaltene Konzentrat auf das 100-fache verdünnt.

Parenterale toxikologische Untersuchungen
Die Untersuchungen der akuten Toxizität wurde nach den Regeln für das Eintragen eines Arzneimittels ins Arzneibuch durchgeführt.

Das Mittel ist zur lokalen Behandlung von verbrannten Hautflächen, bzw. von Hautflächen ohne Epithelschicht geeignet. Im Laufe der pharmakologischen präklinischen Untersuchungen wurden mit dem Mittel 120 Kaninchen, 40 Ratten und 50 Meerschweinchen mit Brandwunden 3 bis 4 Wochen behandelt. Innerhalb dieser Zeit konnte an den Tieren keine auf toxische Wirkung hinweisende krankhafte Veränderung wahrgenommen werden, was als Grundlage der Untersuchung der lokalen Toxizität gelten kann. Obwohl das Arzneimittel nicht absorbiert wird und ausschließlich zur lokalen Behandlung dient, wurden auch die parenteralen Untersuchungen vorgenommen. Da bei der parenteralen Verabreichung mit der Wirkung des Alkohols gerechnet werden muß, wurde für die Untersuchungen das Mittel eingedampft und der Rückstand mit 1 %iger HaHCO$_3$-Lösung bei pH 8,2 erneut gelöst und die Lösung sterilfiltriert.

Die Dosis wurde auf Grund des Gehaltes an Trockensubstanz bestimmt, die maximale Dosis war bei Mäusen 0,5 ml, bei Kaninchen 5,0 ml.

Bei den zu untersuchenden Dosen war der Ausgangspunkt die Menge, die in der Humantherapie an einem Tag maximal auf die Haut des Patienten gelangen kann. Bei Annahme eines Durchschnittsgewichtes von 60 kg und 500 ml Mittel ergibt das 3,0 mg/kg als Tagesdosis, die ausschließlich auf die Hautoberfläche gelangt.

Parenterale Untersuchung der akuten Toxizität

| Tier | Anzahl | Appl. | mg/kg | wievielfaches der Humandosis | Beobach- tungszeit | Ergebnis |
|---|---|---|---|---|---|---|
| Maus | 12 | i.p. | 38,5 | 13× | 2 Monate | Ø |
| Maus | 12 | i.p. | 77,0 | 26× | 2 Monate | 2 am 23. Tag |
| Kaninchen | 12 | i.v. | 8,0 | 2,7× | 2 Wochen bis 2 Monate | Ø |
| Kaninchen | 5 | i.v. | 20,0 | 6,7× | " | Ø |
| Kaninchen | 5 | i.v. | 100,0 | 33× | " | Ø |

Aus der Tabelle ist ersichtlich, daß das Mehrfache der maximal auf die menschliche Haut gelangenden Dosis weder für Mäuse noch für Kaninchen toxisch ist.

Anschließend wurde die $LD_{50}$ bestimmt. Dazu wurden Mäusen des Stammes CLFP LATI i.v. mit dem eingedampften, in $NaHCO_3$-Lösung wieder aufgelösten Mittel geimpft, jede Gruppe umfaßt 20 Tiere. Bei statistischer Auswertung der erhaltene Ergebnisse wurde gefunden, daß der $LD_{50}$-Wert $196,0 \pm 36$ mg/kg beträgt, d.h. das 50fache der ganztägigen menschlichen Dosis.

Behandlung

Die Lösung gemäß Beispiel 1 (nachfolgend: "Mittel") wurde zur Behandlung von 98 mit Verbrennungswunden ins Krankenhaus eingelieferten Kindern angewendet. 25% der Kranken hatten Verbrennungen dritten Grades.

Behandlungsweise:

Das Mittel wurde aus etwa 20 cm Entfernung direkt auf die verletzte Fläche gesprüht, und zwar reichlich, bis zum Abtropen. Wo dazu die Möglichkeit bestand, wurde-statt des als Erste Hilfe empfohlenen Kühlens mit kaltem Wasser-das Mittel auch zum Wärmeentzug verwendet. Bei Brandwunden sehr großer Ausdehnung wurden vor der Behandlung schmerzstillende Mittel verabreicht. Vor der Behandlung wurden vorhandene Blasen entfernt, Salben- und sonstige Reste ebenso.

Die erste Behandlung wurde so lange fortgesetzt, bis der Patient schmerzfrei war (innerhalb von 30 bis 40 Minuten 6 bis 8 Sprühbehandlungen). Später wurde täglich 3 bis 4 mal bzw. täglich einmal behandelt. Während der ganzen Zeit kamen die kranken Flächen mit Wasser oder wäßrigen Lösungen nicht in Berührung.

Verglichen mit den bekannten Methoden der Behandlung (z.B. Sulfodiazin-Silbernitrat-Salbe, Mercurochromlösung) weist die erfindungsgemäße Behandlung folgende Vorteile auf:

1. Die durchschnittliche Dauer des Krankenhausaufenthaltes sank um 28%.

2. Bei einem großen Teil der Kranken konnte nach einigen Tagen die Behandlung als ambulante Behandlung fortgesetzt werden.

3. Wurde das Mittel sofort eingesetzt, so waren im allgemeinen keine plastischen Operationen erforderlich.

4. Wurde das Mittel bereits bei der Ersten Hilfe eingestzt, so war die Kompensierung des Brandschocks leichter. Nach 24 bis 36 Stunden bildete sich Grind, der den Verlust an Plasma bzw. Feuchtigkeit sowie Infektionen verhinderte.

5. 25 bis 30% weniger Humanplasma, Flüssigkeit und kalorischer Ersatz waren erforderlich als bei anderen Verfahren.

6. Wird sofort mit dem Mittel behandelt, so ist weder parenterale noch lokale Behandlung mit Antibiotika erforderlich. Wurde zuerst mit etwas anderem behandelt, so genügte nach der Umstellung auf das Mittel eine lokale Behandlung mit Antibiotikum.

7. Brandsepsis, Leber- oder Nierenschäden traten auch bei den Fällen dritten Grades nicht auf.

8. Die Untersuchung der lebenswichtigen Organe (Nieren, Leber) und des Bluts ziegten, daß weder eine giftige noch eine allergische Nebenwirkung vorliegt.

9. Bei offener Behandlung (ohne Verband) trat die Bildung von Grind immer ein. Unter dem Grind sammelte sich niemals Flüssigkeit an.

10. Die Behandlung selbst verursachte nur in den ersten 24 Stunden 1 bis 2 Minuten Schmerzen. Nach der Bildung des Schorfs ist die Behandlung schmerzlos und viele Kranke (größere Kinder) konnten sich selbst behandeln.

12

# EP 0 103 878 B1

11. Im Vergleich mit den bekannten Behandlungsmethoden sind die Krankenhauskosten etwa 40% geringer.

**Patentansprüche für die Vertragsstaaten: BE CH DE FR GB IT LI NL**

1. Präparat zur Behandlung von Wunden der Hautoberfläche, dadurch gekennzeichnet, daß es in 100 ml $C_{2-4}$-Alkanollösung

bis 20 mg pflanzliche Gerbstoffe,
bis 50 mg Zucker,
0,3—6 mg Verbindungen vom Anthocyan- und/oder Flavontyp,
0,1—6 mg Pectin,
bis 6 mg Pflanzenwachs und
0,005—0,1 mg ätherische Öle enthält,

jedoch mit der Ausnahme, daß bei einem

| Gerbstoffgehalt | von 5—20 mg, |
| der Gehalt an Zucker | nicht 5—15 mg, |
| an Anthocyan, einer Verbindung vom | |
| Flavontyp und/oder Pectin | nicht 0,5—6 mg |
| und an Pflanzenwachs betragen darf. | nicht 0,5—6 mg |

2. Präparat nach Anspruch 1, dadurch gekennzeichnet, daß es in 100 ml $C_{2-4}$-Alkanollösung
1—20 mg pflanzliche Gerbstoffe,
1—50 mg Zucker,
0,5—6 mg Verbindungen vom Anthocyan- und/oder Flavontyp,
0,5—6 mg Pectin,
0,1—6 mg Pflanzenwachs und
0,01—0,1 mg ätherische Öle

enthält.
3. Präparat nach Anspruch 1 oder 2, dadurch gekennzeichnet, daß es als zusätzliche Komponenten 0,1—1 mg Vitamine und anorganische Salze enthält.
4. Präparat nach Anspruch 1, 2 oder 3, dadurch gekennzeichnet, daß
es als pflanzliche Gerbstoffe Brenzcatechin, Tannin, Gallussäure, Digallussäurer und/oder Pentadigalloyl-glucose,
als Zucker Glucose, Fructose, Ribose, Arabinose, Rhamnose und/oder Xylose,
als Verbindungen vom Flavontyp Quercetin,
als ätherisches Öl Geraniol 40—90%, Nerol 4—15%, Citronellol 10—50%, Eugenol, Linalool bzw. die entsprechenden Aldehyde,
als Vitamine die Vitamine A, $B_1$, $B_2$, $B_6$, $B_{12}$, C, K und/oder P,
als Spurenelemente Mangan, Magnesium, Calcium, Eisen, Zink, Kupfer, Molybdän, Cobalt und/oder Nickel,
als Pflanzenhormone Auxin und/oder Kinetin in ng-Mengen,
als Enzyme mit oxydativer Wirkung Peroxydase und/oder Katalase und
als anorganische Salze Phosphate und/oder Chloride enthält.
5. Präparat nach Anspruch 1, 2, 3 oder 4, gekennzeichnet durch 70—100 %iges, vorzugsweise 96 %iges, Äthanol als Lösungsmittel.
6. Verfahren zur Herstellung von Präparaten zur Behandlung von Wunden der Hautoberfläche, nach Anspruch 1, dadurch gekennzeichnet, daß man

bis 20 mg pflanzliche Gerbstoffe,
bis 50 mg Zucker,
0,3—6 mg Verbindungen vom Anthocyan und/oder Flavontyp,
0,1—6 mg Pectin,
bis 6 mg Pflanzenwachs und
0,005—0,1 mg ätherische Öle

auf einmal oder verteilt in $C_{2-4}$-Alkanol zu 100 ml Lösung löst, jedoch mit der Ausnahme, daß bei Verwendung einer

| Gerbstoffmenge | von 5—20 mg, |
| die verwendete Menge an Zucker | nicht 5—15 mg und |
| an Anthocyan, einer Verbindung vom | |
| Flavontyp und/oder Pectin | nicht 0,5—6 mg |
| betragen darf. | |

7. Verfahren nach Anspruch 6, dadurch gekennzeichnet, daß man

1—20 mg pflanzliche Gerbstoffe,
1—50 mg Zucker,
0,5—6 mg Verbindungen vom Anthocyan- und/oder Flavontyp,
0,5—6 mg Pectin,
0,1—6 mg Pflanzenwachs und
0,01—0,1 mg ätherische Öle

auf einmal oder verteilt in $C_2$—$C_4$-Alkanol zu 100 ml Lösung löst.

8. Verfahren nach Anspruch 6 oder 7, dadurch gekennzeichnet, daß man 0,1—1 mg Vitamine und anorganische Salze als zusätzliche Komponenten verwendet.

9. Verfahren nach Anspruch 6, 7 oder 8, dadurch gekennzeichnet, daß man

als pflanzliche Gerbstoffe Branzcatechin, Tannin, Gallussäure, Digallussäure und/oder Pentadigalloyl-glucose,
als Zucker Glucose, Fructose, Ribose, Arabinose, Rhamnose und/oder Xylose,
als Verbindungen vom Anthocyantyp Anthocyan und/oder Cyanidin,
als Verbindungen vom Flavontyp Quercetin,
als ätherisches Öl Geraniol 40—90%, Nerol 4—15%, Citronellol 10—50%, Eugenol, Linalool bzw. die entsprechenden Aldehyde,
als Vitamine die Vitamine A, $B_1$, $B_2$, $B_6$, $B_{12}$, C, K und/oder P,
als Spurenelemente Mangan, Magnesium, Calcium, Eisen, Zink, Kupfer, Molybdän, Cobalt und/oder Nickel,
als Pflanzenhormone Auxin und/oder Kinetin in ng-Mengen,
als Enzyme mit oxydativer Wirkung Peroxydase und/oder Katalase und
als anorganische Salze Phosphate und/oder Chloride verwendet.

10. Verfahren nach Anspruch 6, 7, 8 oder 9, dadurch gekennzeichnet, daß man als Lösungsmittel 70—100 %iges, vorzugsweise 96 %iges, Äthanol verwendet.

**Patentansprüche für die Vertragsstaaten: LU SE**

1. Präparat zur Behandlung von Wunden der Hautoberfläche, dadurch gekennzeichnet, daß es in 100 ml $C_{2-4}$-Alkanollösung

bis 20 mg pflanzliche Gerbstoffe,
bis 50 mg Zucker,
0,3—6 mg Verbindungen vom Anthocyan- und/oder Flavontyp,
0,1—6 mg Pectin,
bis 6 mg Pflanzenwachs und
0,005—0,1 mg ätherische Öle

enthält.

2. Präparat nach Anspruch 1, dadurch gekennzeichnet, daß es in 100 ml $C_{2-4}$-Alkanollösung

1—20 mg pflanzliche Gerbstoffe,
1—50 mg Zucker,
0,5—6 mg Verbindungen vom Anthocyan- und/oder Flavontyp,
0,5—6 mg Pectin,
0,1—6 mg Pflanzenwachs und
0,01—0,1 mg ätherische Öle

enthält.

3. Präparat nach Anspruch 1 oder 2, dadurch gekennzeichnet, daß es als zusätzliche Komponenten

0,1—1 mg Vitamine und anorganische Salze

enthält.

4. Präparat nach Anspruch 1, 2 oder 3, dadurch gekennzeichnet, daß es als pflanzliche Gerbstoffe, Brenzcatechin, Tannin, Gallussäure, Digallussäure und/oder Pentadigalloyl-glucose,

## EP 0 103 878 B1

als Zucker Glucose, Fructose, Ribose, Arabinose, Rhamnose und/oder Xylose,
als Verbindungen vom Anthocyantyp Anthocyan und/oder Cyanidin,
als Verbindungen vom Flavontyp Quercetin,
als ätherisches Öl Geraniol 40—90%, Nerol 4—15%, Citronellol 10—50%, Eugenol, Linalool bzw. die entsprechenden Aldehyde,
als Vitamine die Vitamine A, $B_1$, $B_2$, $B_6$, $B_{12}$, C, K und/oder P,
als Spurenelemente Mangan, Magnesium, Calcium, Eisen, Zink, Kupfer, Molybdän, Cobalt und/oder Nickel,
als Pflanzenhormone Auxin und/oder Kinetin in ng-Mengen,
als Enzyme mit oxydativer Wirkung Peroxydase und/oder Katalse und
als anorganische Salze Phosphat und/oder Chloride

enthält.

5. Präparat nach Anspruch 1, 2, 3 oder 4, dadurch gekennzeichnet durch 70—100 %iges, vorzugsweise 96 %iges, Äthanol als Lösungsmittel.

6. Verfahren zur Herstellung von Präparaten zur Behandlung von Wunden der Hautoberfläche nach Anspruch 1, dadurch gekennzeichnet, daß man

bis 20 mg pflanzliche Gerbstoffe,
bis 50 mg Zucker,
0,3—6 mg Verbindungen vom Anthocyan- und/oder Flavontyp,
0,1—6 mg Pectin,
bis 6 mg Pflanzenwachs und
0,005—0,1 mg ätherische Öle

auf einmal oder verteilt in $C_{2-4}$-Alkanol zu 100 ml Lösung löst.

7. Verfahren nach Anspruch 6, dadurch gekennzeichnet, daß man

1—20 mg pflanzliche Gerbstoffe,
1—50 mg Zucker,
0,5—6 mg Verbindungen vom Anthocyan- und/oder Flavontyp,
0,1—6 mg Pectin,
0,1—6 mg Pflanzenwachs und
0,01—0,1 mg ätherische Öle

auf einmal oder verteilt in $C_{2-4}$-Alkanol zu 100 ml Lösung löst.

8. Verfahren nach Anspruch 6 oder 7, dadurch gekennzeichnet, daß man

0,1—1 mg Vitamine und anorganische Salze

als zusätzliche Komponenten verwendet.

9. Verfahren nach Anspruch 6, 7 oder 8, dadurch gekennzeichnet, daß man
als pflanzliche Gerbstoffe Branzcatechin, Tannin, Gallussäure, Digallussäure und/oder Pentadigalloyl-glucose,
als Zucker, Glucose, Fructose, Ribose, Arabinose, Rhamnose und/oder Xylose,
als Verbindungen vom Anthocyantyp Anthocyan und/oder Cyanidin,
als Verbindungen vom Flavontyp Quercetin,
als ätherisches Öl Geraniol 40—90%, Nerol 4—15%, Citronellol 10—50%, Eugenol, Linalool bzw. die entsprechenden Aldehyde,
als Vitamine die Vitamine A, $B_1$, $B_2$, $B_6$, $B_{12}$, C, K und/oder P,
als Spurenelemente Mangan, Magnesium, Calcium, Eisen, Zink, Kupfer, Molybdän, Cobalt und/oder Nickel,
als Pflanzenhormone Auxin und/oder Kinetin in ng-Mengen,
als Enzyme mit oxydativer Wirkung Peroxydase und/oder Katalase und
als anorganische Salze Phosphate und/oder Chloride verwendet.

10. Verfahren nach Anspruch 6, 7, 8 oder 9, dadurch gekennzeichnet, daß man als Lösungsmittel 70—100 %iges, vorzugsweise 96 %iges, Äthanol verwendet.

**Patentansprüche für den Vertragsstaat: AT**

1. Verfahren zur Herstellung von Präparaten zur Behandlung von Wunden der Hautoberfläche, dadurch gekennzeichnet, daß man

bis 20 mg pflanzliche Gerbstoffe,
bis 50 mg Zucker,

15

03,—6 mg Verbindungen vom Anthocyan und/oder Flavontyp,
0,1—6 mg Pectin,
bis 6 mg Pflanzenwachs und
0,005—0,1 mg ätherische Öle

auf einmal oder verteilt in $C_{2-4}$-Alkanol zu 100 ml Lösung löst, jedoch mit der Ausnahme, daß bei Verwendung einer

| | |
|---|---|
| Gerbstoffmenge | von 5—20 mg, |
| die verwendete Menge an Zucker | nicht 5—15 mg und |
| an Anthocyan, einer Verbindung vom | |
| Flavontyp und/oder Pectin | nicht 0,5—6 mg |
| betragen darf. | |

2. Verfahren nach Anspruch 1, dadurch gekennzeichnet, daß man

1—20 mg pflanzliche Gerbstoffe,
1—50 mg Zucker,
0,5—6 mg Verbindungen vom Anthocyan- und/oder Flavontyp,
0,5—6 mg Pectin,
0,1—6 mg Pflanzenwachs und
0,01—0,1 mg ätherische Öle

auf einmal oder verteilt in $C_{2-4}$-Alkanol zu 100 ml Lösung löst.
3. Verfahren nach Anspruch 1 oder 2, dadurch gekennzeichnet, daß man

0,1—1 mg Vitamine und anorganische Salze

als zusätzliche Komponenten verwendet.
4. Verfahren nach Anspruch 1, 2 oder 3, dadurch gekennzeichnet, daß man
als pflanzliche Gerbstoffe Branzcatechin, Tannin, Gallussäure, Digallussäure und/oder Pentadigalloyl-glucose,
als Zucker Glucose, Fructose, Ribose, Arabinose, Rhamnose und/oder Xylose,
als Verbindungen vom Anthocyantyp Anthocyan und/oder Cyanidin,
als Verbindungen vom Flavontyp Quercetin,
als ätherisches Öl Geraniol 40—90%, Nerol 4—15%, Citronellol 10—50%, Eugenol, Linalool bzw. die entsprechenden Aldehyde,
als Vitamine die Vitamine A, $B_1$, $B_2$, $B_6$, $B_{12}$, C, K und/oder P,
als Spurenelemente Mangan, Magnesium, Calcium, Eisen, Zink, Kupfer, Molybdän, Cobalt und/oder Nickel,
als Pflanzenhormone Auxin und/oder Kinetin in ng-Mengen,
als Enzyme mit oxydativer Wirkung Peroxydase und/oder Katalase und
als anorganische Salze Phosphate und/oder Chloride verwendet.
5. Verfahren nach Anspruch 1, 2, 3 oder 4, dadurch gekennzeichnet, daß man als Lösungsmittel 70—100 %iges, vorzugsweise 96 %iges, Äthanol, verwendet.

**Revendications pour les Etats contractants: BE CH DE FR GB IT LI NL**

1. Préparation pour le traitement des plaies de la surface de la peau, caractérisée en ce qu'elle contient dans 100 ml d'une solution d'un alcanol en $C_{2-4}$

jusqu'à 20 mg de tannins végétaux,
jusqu'à 50 mg de sucre,
0,3—6 mg de composés du type anthocyane et/ou du type flavone,
0,1—6 mg de pectine,
jusqu'à 6 mg de cire végétale et
0,005—0,1 mg d'huiles éthérées,

avec toutefois cette exception que pour une

| | |
|---|---|
| teneur en tannin | de 5—20 mg, |
| la teneur en sucre ne doit pas être située entre | 5—15 mg, |
| les teneurs en anthocyane, en un composé du type flavone et/ou en pectine ne doivent pas être situées entre | 0,5—6 mg, |
| et la teneur en cire végétale entre | 0,5—6 mg. |

2. Préparation selon la revendication 1, caractérisée en ce qu'elle contient dans 100 ml d'une solution d'un alcanol en $C_{2-4}$

1—20 mg de tannins végétaux,
1—50 mg de sucre,
0,5—6 mg de composés du type anthocyane et/ou du type flavone,
0,5—6 mg de pectine,
0,1—6 mg de cire végétale, et
0,01—0,1 mg d'huiles éthérées.

3. Préparation selon la revendication 1 ou 2, caractérisée en ce qu'elle contient, comme composants additionnels, 0,1—1 mg de vitamines et de sels minéraux.

4. Préparation selon l'une des revendications 1, 2 ou 3, caractérisée en ce qu'elle contient comme tannins végétaux: la pyrocatéchine, le tannin, l'acide gallique, l'acide digallique et/ou le pentadigalloyl-glucose,

comme sucres: le glucose, le fructose, le ribose, l'arabinose, le rhamnose et/ou le xylose,
comme composés du type anthocyane: l'anthocyane et/ou la cyanidine,
comme composé du type flavone: la quercétine,
comme huiles éthérées: 40—90% de géraniol, 4—15% de nérol, 10—50% de citronellol, l'eugénol, le linalol ou les aldéhydes correspondants,
comme vitamines: les vitamines A, $B_1$, $B_2$, $B_6$, $B_{12}$, C, K et/ou P,
comme oligo-éléments: le manganèse, le magnésium, le calcium, le fer, le zinc, le cuivre, le molybdène, le cobalt et/ou le nickel,
comme hormones végétales: l'auxine et/ou la cinétine en quantités en ng,
comme enzymes ayant une action oxydante: la peroxydase et/ou la catalase et
comme sels minéraux: des phosphates et/ou des chlorures.

5. Préparation selon l'une des revendications 1, 2, 3 ou 4, caractérisée en ce qu'elle contient de l'éthanol à 70—100%, de préférence à 96%, comme solvant.

6. Procédé pour l'obtention des préparations pour le traitement des plaies de la surface de la peau, selon la revendication 1, caractérisé en ce que l'on dissout jusqu'à 20 mg de tannins végétaux,

jusqu'à 50 mg de sucre,
0,3—6 mg de composés du type anthocyane et/ou du type flavone,
0,1—6 mg de pectine,
jusqu'à 6 mg de cire végétale et
0,005—0,1 mg d'huiles éthérées

en une ou plusieurs fois dans un alcanol en $C_{2-4}$ pour faire 100 ml de solution, avec toutefois cette exception que, lorsque l'on utilise

| | |
|---|---|
| une quantité de tannin de | 5—20 mg, |
| la quantité de sucre utilisée ne doit pas être située entre | 5—15 mg |
| et les quantités d'anthocyane, d'un composé du type flavone et/ou de la pectine utilisées ne doivent pas être situées entre | 0,5—6 mg. |

7. Procédé selon la revendication 6, caractérisé en ce que l'on dissout

1—20 mg de tannins végétaux,
1—50 mg de sucre,
0,5—6 mg de composés du type anthocyane et/ou du type flavone,
0,5—6 mg de pectine,
0,1—6 mg de cire végétale et
0,01—0,1 mg d'huiles éthérées,

en une ou plusieurs fois dans un alcanol en $C_2$—$C_4$ pour faire 100 ml de solution.

8. Procédé selon la revendication 6 ou 7, caractérisé en ce que l'on utilise comme composants additionnels, 0,1—1 mg de vitamines et de sels minéraux.

9. Procédé selon l'une des revendications 6, 7 ou 8, caractérisé en ce que l'on utilise

comme tannins végétaux: la pyrocatéchine, le tannin, l'acide gallique, l'acide digallique et/ou le pentadigalloyl-glucose,
comme sucres: le glucose, le fructose, le ribose, l'arabinose, le rhamnose et/ou le xylose,
comme composés du type anthocyane: l'anthocyane et/ou la cyanidine,
comme composé du type flavone: la quercétine,

EP 0 103 878 B1

comme huiles éthérées: 40—90% de géraniol, 4—15% de nérol, 10—50% de citronellol, l'eugénol, le linalol ou les aldéhydes correspondants,

comme vitamines: les vitamines A, $B_1$, $B_2$, $B_6$, $B_{12}$, C, K et/ou P,

comme oligo-éléments: le manganèse, le magnésium, le calcium, le fer, le zinc, le cuivre, le molybdène, le cobalt et/ou le nickel,

comme hormones végétales: l'auxine et/ou la cinétine en quantités en ng,

comme enzymes ayant une action oxydante: la peroxydase et/ou la catalase et

comme sels minéraux: des phosphate et ou des chlorures.

10. Procédé selon l'une des revendications 6, 7, 8 ou 9, caractérisé en ce que l'on utilise comme solvant de l'éthanol à 70—100%, de préférence à 96%.

**Revendications pour les Etats Contractants: LU SE**

1. Préparation pour le traitement des plaies de la surface de la peau, caractérisée en ce qu'elle contient dans 100 ml d'une solution d'un alcanol en $C_{2-4}$

jusqu'à 20 mg de tannins végétaux,
jusqu'à 50 mg de sucre,
0,3—6 mg de composés du type anthocyane et/ou du type flavone,
0,1—6 mg de pectine,
jusqu'à 6 mg de cire végétale et
0,005—0,1 mg d'huiles éthérées.

2. Préparation selon la revendication 1, caractérisée en ce qu'elle contient dans 100 ml d'une solution d'un alcanol en $C_{2-4}$

1—20 mg de tannins végétaux,
1—50 mg de sucre,
0,5—6 mg de composés du type anthocyane et/ou du type flavone,
0,5—6 mg de pectine,
0,1—6 mg de cire végétale et
0,01—0,1 mg d'huiles éthérées.

3. Préparation selon la revendication 1 ou 2, caractérisée en ce qu'elle contient, comme composants additionnels, 0,1—1 mg de vitamines et de sels minéraux.

4. Préparation selon l'une des revendications 1, 2 ou 3, caractérisée en ce qu'elle contient

comme tannins végétaux: la pyrocatéchine, le tannin, l'acide gallique, l'acide digallique et/ou le pentadigalloyl-glucose,

comme scures: le glucose, le fructose, le ribose, l'arabinose, le rhamnose et/ou le xylose,

comme composés du type anthocyane: l'anthocyane et/ou la cyanidine,

comme composé du type flavone: la quercétine,

comme huiles éthérées: 40—90% de géraniol, 5—15% de nérol, 10—50% de citronellol, l'eugénol, le linalol ou les aldéhydes correspondants,

comme vitamines: les vitamines A, $B_1$, $B_2$, $B_6$, $B_{12}$, C, K et/ou P,

comme oligo-éléments: le manganèse, le magnésium, le calcium, le fer, le zinc, le cuivre, le molybdène, le cobalt et/ou le nickel,

comme hormones végétales: l'auxine et/ou la cinétine en quantités en ng,

comme enzymes ayant une action oxydante: la peroxydase et/ou la catalase et

comme sels minéraux: des phosphates et/ou des chlorures.

5. Préparation selon l'une des revendications 1, 2, 3 ou 4, caractérisée en ce qu'elle contient de l'éthanol à 70—100%, de préférence à 96%, comme solvant.

6. Procédé par l'obtention de préparations pour le traitement des plaies de la surface de la peau, selon la revendication 1, caractérisé en ce que l'on dissout

jusqu'à 20 mg de tannins végétaux,
jusqu'à 50 mg de sucre,
0,3—6 mg de composés du type anthocyane et/ou du type flavone,
0,1—6 mg de pectine,
jusqu'à 6 mg de cire végétale et
0,005—0,1 mg d'huiles éthérées,
en une ou plusieurs fois dans un alcanol en $C_{2-4}$ pour faire 100 ml de solution.

7. Procédé selon la revendication 6, caractérisé en ce que l'on dissout
1—20 mg de tannins végétaux,

18

1—50 mg de sucre,
0,5—6 mg de composés du type anthocyane et/ou du type flavone,
0,1—6 mg de pectine,
0,1—6 mg de cire végétale et
0,01—0,1 mg d'huiles éthérées,

en une ou plusieurs fois dans un alcanol en $C_{2-4}$ pour faire 100 ml de solution.

8. Procédé selon la revendication 6 ou 7, caractérisé en ce que l'on utilise, comme composants additionnels, 0,1—1 mg de vitamines et de sels minéraux.

9. Procédé selon l'une des revendications 6, 7 ou 8, caractérisé en ce que l'on utilise
comme tannins végétaux: la pyrocatéchine, le tannin, l'acide gallique, l'acide digallique et/ou le pentadigalloyl-glucose,
comme sucres: le glucose, le fructose, le ribose, l'arabinose, le rhamnose et/ou le xylose,
comme composés du type anthocyane: l'anthocyane et/ou la cyanidine,
comme composé du type flavone: la quercétine,
comme huiles éthérées: 40—90% de géraniol, 4—15% de nérol et 10—50% de citronellol, l'eugénol, le linalol ou les aldéhydes correspondants,
comme vitamines: les vitamines A, $B_1$, $B_2$, $B_6$, $B_{12}$, C, K et/ou P,
comme oligo-éléments: le manganèse, le magnésium, le calcium, le fer, le zinc, le cuivre, le molybdène, le cobalt et/ou le nickel,
comme hormones végétales: l'auxine et/ou la cinétine en quantités en ng,
comme enzymes ayant une action oxydante: la peroxydase et/ou la catalase et
comme sels minéraux: des phosphates et/ou des chlorures.

10. Procédé selon l'une des revendications 6, 7, 8 ou 9, caractérisé en ce que l'on utilise, comme solvant, de l'éthanol à 70—100%, de préférence à 96%.

**Revendications pour l'Etat contractant: AT**

1. Procédé pour l'obtention de préparations pour le traitement des plaies de la surface de la peau, caractérisé en ce que l'on dissout

jusqu'à 20 mg de tannins végétaux,
jusqu'à 50 mg de sucre,
0,3—6 mg de composés du type anthocyane et/ou du type flavone,
0,1—6 mg de pectine,
jusqu'à 6 mg de cire végétale et
0,005—0,1 mg d'huiles éthérés

en une ou plusieurs fois dans un alcanol en $C_{2-4}$ pour faire 100 ml de solution, avec toutefois cette exception que, lorsque l'on utilise

| | |
|---|---|
| une quantité de tannin de | 5—20 mg, |
| la quantité de sucre utilisée ne doit pas être située entre | 5—15 mg |
| et les quantités d'anthocyane, d'un composé du type flavone et/ou de la pectine utilisées ne doivent pas être situées entre | 0,5—6 mg. |

2. Procédé selon la revendication 1, caractérisé en ce que l'on dissout

1—20 mg de tannins végétaux,
1—50 mg de sucre,
0,5—6 mg de composés du type anthocyane et/ou du type flavone,
0,5—6 mg de pectine,
0,1—6 mg de cire végétale et
0,01—0,1 mg d'huiles éthérées,

en une ou plusieurs fois dans un alcanol en $C_{2-4}$ pour faire 100 ml de solution.

3. Procédé selon la revendication 1 ou 2, caractérisé en ce que l'on utilise comme composants additionnels, 0,1—1 mg de vitamines et de sels minéraux.

4. Procédé selon l'une des revendications 1, 2 ou 3, caractérisé en ce que l'on utilise
comme tannins végétaux: la pyrocatéchine, le tannin, l'acide gallique, l'acide digallique et/ou le pentadigalloyl-glucose,
comme sucres: le glucose, le fructose, le ribose, l'arabinose, le rhamnose et/ou le xylose,
comme composés du type anthocyane: l'anthocyane et/ou la cyanidine,

comme composé du type flavone: la quercétine,

comme huiles éthérées: 40—90% de géraniol, 4—15% de nérol et 10—50% de citronellol, l'eugénol, le linalol ou les aldéhydes correspondants,

comme vitamines: les vitamines A, $B_1$, $B_2$, $B_6$, $B_{12}$, C, K et/ou P,

comme oligo-éléments: le manganèse, le magnésium, le calcium, le fer, le zinc, le cuivre, le molybdène, le cobalt et/ou le nickel,

comme hormones végétales: l'auxine et/ou la cinétine en quantités en ng,

comme enzymes ayant une action oxydante: la peroxydase et/ou la catalase et

comme sels minéraux: des phosphates et/ou des chlorures.

5. Procédé selon l'une des revendications 1, 2, 3 ou 4, caractérisé en ce que l'on utilise comme solvant de l'éthanol à 70—100%, de préférence à 96%.

## Claims for the Contracting States: BE CH DE FR GB IT LI NL

1. Preparation for treating wounds on the surface of the skin, characterised in that it contains, in 100 ml of $C_{2-4}$ alkanol solution,

up to 20 mg of vegetable tannins,
up to 50 mg of sugar,
0.3 to 6 mg of compounds of the anthocyan and/or flavone type,
0.1 to 6 mg of pectin,
up to 6 mg of vegetable wax and
0.005 to 0.1 mg of ethereal oils,

with the exception that, in the event of a tannin

| | |
|---|---|
| content of | 5 to 20 mg, |
| the sugar content should not be | 5 to 15 mg |
| the content of anthocyan, a compound of the flavone type and/or pectin should not be | 0.5 to 6 mg |
| and the content of vegetable wax should not be | 0.5 to 6 mg. |

2. Preparation according to claim 1, characterised in that it contains, in 100 ml of $C_{2-4}$ alkanol solution,

1 to 20 mg of vegetable tannins,
1 to 50 mg of sugar,
0.5 to 6 mg of compounds of the anthocyan and/or flavone type
0.5 to 6 mg of pectin,
0.1 to 6 mg of vegetable wax and
0.01 to 0.1 mg of ethereal oils.

3. Preparation according to claim 1 or 2, characterised in that it contains as additional components

0.1 to 1 mg of vitamins and inorganic salts.

4. Preparation according to claim 1, 2 or 3, characterised in that it contains as vegetable tannins pyrocatechol, tannin, gallic acid, digallic acid and/or pentadigalloyl glucose,

as sugar: glucose, fructose, ribose, arabinose, rhamnose and/or xylose,
as compounds of the anthocyan type: anthocyan and/or cyanidine,
as compounds of the flavone type: quercetine,
as ethereal oil: geraniol 40 to 90%, nerol 4 to 15%, citronellol 10 to 50%, eugenol, linalool or the corresponding aldehydes,
as vitamins: vitamins A, $B_1$, $B_2$, $B_6$, $B_{12}$, C, K and/or P,
as trace elements: manganese, magnesium, calcium, iron, zinc, copper, molybdenum, cobalt and/or nickel,
as plant hormones auxin and/or kinetin in ng amounts,
as enzymes with an oxidative activity: peroxidase and/or catalase and
as inorganic salts: phosphates and/or chlorides.

5. Preparation according to claim 1, 2, 3 or 4, characterised in that it contains 70 to 100%, preferably 96%, ethanol as solvent.

6. Process for producing preparations for treating wounds on the surface of the skin according to claim 1, characterised in that

up to 20 mg of vegetable tannins,
up to 50 mg of sugar,
0.3 to 6 mg of compounds of the anthocyan and/or flavone type,
0.1 to 6 mg of pectin,
up to 6 mg of vegetable wax and
0.005 to 0.1 mg of ethereal oils are dissolved all at once or in separate batches in $C_{2-4}$ alkanol per 100 ml of solution, but with the exception that if a quantity of tannins of 5 to 20 mg is used, the quantity of sugar used must not be 5 to 15 mg and the quantity of anthocyan, a compound of the flavone type and/or pectin must not be 0.5 to 6 mg.

7. Process according to claim 6, characterised in that

1 to 20 mg of vegetable tannins,
1 to 50 mg of sugar,
0.5 to 6 mg of compounds of the anthocyan and/or flavone type,
0.5 to 6 mg of pectin,
0.1 to 6 mg of vegetable wax and
0.01 to 0.1 mg of ethereal oils are dissolved all at once or distributed in $C_{2-4}$ alkanol per 100 ml of solution.

8. Process according to claim 6 or 7, characterised in that 0.1 to 1 mg of vitamins and inorganic salts are used as additional components.

9. Process according to claim 6, 7 or 8, characterised in that
the vegetable tannins used are pyrocatechol, tannin, gallic acid, digallic acid and/or pentadigalloyl glucose,
the sugar used is glucose, fructose, ribose, arabinose, rhamnose and/or xylose,
the compounds of the anthocyan type used are anthocyan and/or cyanidine,
the compound of the flavone type used is quercetine,
the ethereal oil used is geraniol 40 to 90%, nerol 4 to 15%, citronellol 10 to 50%, eugenol, linalool or the corresponding aldehydes,
the vitamins used are vitamins $A$, $B_1$, $B_2$, $B_6$, $B_{12}$, $C$, $K$ and/or $P$,
the trace elements used are manganese, magnesium, calcium, iron, zinc, copper, molybdenum, cobalt and/or nickel,
the plant hormones used are auxin and/or kinetin in ng amounts,
the enzymes with an oxidative activity used are peroxidase and/or catalase, and
the inorganic salts used are phosphates and/or chlorides.

10. Process according to claim 6, 7, 8 or 9, characterised in that the solvent used is 70 to 100%, preferably 96%, ethanol.

**Claims for the Contracting States: LU SE**

1. Preparation for treating wounds on the surface of the skin, characterised in that it contains, in 100 ml of $C_{2-4}$ alkanol solution,

up to 20 mg of vegetable tannins,
up to 50 mg of sugar,
0.3 to 6 mg of compounds of the anthocyan and/or flavone type,
0.1 to 6 mg of pectin,
up to 6 mg of vegetable wax and
0.005 to 0.1 mg of ethereal oils.

2. Preparation according to claim 1, characterised in that it contains, in 100 ml of $C_{2-4}$ alkanol solution,

1 to 20 mg of vegetable tannins,
1 to 50 mg of sugar,
0.5 to 6 mg of compounds of the anthocyan and/or flavone type,
0.5 to 6 mg of pectin,
0.1 to 6 mg of vegetable wax and
0.01 to 0.1 mg of ethereal oils.

3. Preparation according to claim 1 or 2, characterised in that it contains as additional components

0.1 to 1 mg of vitamins and inorganic salts.

4. Preparation according to claim 1, 2 or 3, characterised in that it contains as vegetable tannins pyrocatechol, tannin, gallic acid, digallic acid and/or pentadigalloyl glucose,

# EP 0 103 878 B1

as sugar: glucose, fructose, ribose, arabinose, rhamnose and/or xylose,

as compounds of the anthocyan type: anthocyan and/or cyanidine,

as compounds of the flavone type: quercetine,

as ethereal oil: geraniol 40 to 90%, nerol 4 to 15%, citronellol 10 to 50%, eugenol, linalool or the corresponding aldehydes,

as vitamins: vitamins A, $B_1$, $B_2$, $B_6$, $B_{12}$, C, K and/or P,

as trace elements: manganese, magnesium, calcium, iron, zinc, copper, molybdenum, cobalt and/or nickel,

as plant hormones auxin and/or kinetin in ng amounts,

as enzymes with an oxidative activity: peroxidase and/or catalase and

as inorganic salts: phosphates and/or chlorides.

5. Preparation according to caim 1, 2, 3 or 4, characterised in that it contains 70 to 100%, preferably 96%, ethanol as solvent.

6. Process for producing preparations for treating wounds on the surface of the skin according to claim 1, characterised in that

up to 20 mg of vegetable tannins,

up to 50 mg of sugar,

0.3 to 6 mg of compounds of the anthocyan and/or flavone type,

0.1 to 6 mg of pectin,

up to 6 mg of vegetable wax and

0.005 to 0.1 mg of ethereal oils are dissolved all at once or in separate batches in $C_{2-4}$ alkanol per 100 ml of solution.

7. Process according to claim 6, characterised in that

1 to 20 mg of vegetable tannins,

1 to 50 mg of sugar,

0.5 to 6 mg of compounds of the anthocyan and/or flavone type,

0.5 to 6 mg of pectin,

0.1 to 6 mg of vegetable wax and

0.01 to 0.1 mg of ethereal oils are dissolved all at once or distributed in $C_{2-4}$ alkanol per 100 ml of solution.

8. Process according to claim 6 or 7, characterised in that 0.1 to 1 mg of vitamins and inorganic salts are used as additional components.

9. Process according to claim 6, 7 or 8, characterised in that

the vegetable tannins used are pyrocatechol, tannin, gallic acid, digallic acid and/or pentadigalloyl glucose,

the sugar used is glucose, fructose, ribose, arabinose, rhamnose and/or xylose,

the compounds of the anthocyan type used are anthocyan and/or cyanidine,

the compound of the flavone type used is quercetine,

the ethereal oil usd is geraniol 40 to 90%, nerol 4 to 15%, citronellol 10 to 50%, eugenol, linalool or the corresponding aldehydes,

the vitamins used are vitamins A, $B_1$, $B_2$, $B_6$, $B_{12}$, C, K and/or P,

the trace elements used are manganese, magnesium, calcium, iron, zinc, copper, molybdenum, cobalt and/or nickel,

the plant hormones used are auxin and/or kinetin in ng amounts,

the enzymes with an oxidative activity used are peroxidase and/or catalase, and

the inorganic salts used are phosphates and/or chlorides.

10. Process according to claim 6, 7, 8 or 9, characterised in that the solvent used is 70 to 100%, preferably 96%, ethanol.

**Claims for the Contracting State: AT**

1. Process for producing preparations for treating wounds on the surface of the skin, characterised in that

up to 20 mg of vegetable tannins,

up to 50 mg of sugar,

0.3 to 6 mg of compunds of the anthocyan and/or flavone type,

0.1 to 6 mg of pectin,

up to 6 mg of vegetable wax and

0.005 to 0.1 mg of ethereal oils are dissolved all at once or in separate batches in $C_{2-4}$ alkanol per 100 ml of solution, but with the exception that if a quantity of tannins of 5 to 20 mg is used, the quantity of sugar used must not be 5 to 15 mg and the quantity of anthocyan, a compound of the flavone type and/or pectin must not be 0.5 to 6 mg.

22

# EP 0 103 878 B1

2. Process according to claim 1, characterised in that

1 to 20 mg of vegetable tannins,
1 to 50 mg of sugar,
0.5 to 6 mg of compounds of the anthocyan and/or flavone type,
0.5 to 6 mg of pectin,
0.1 to 6 mg of vegetable wax and
0.01 to 0.1 mg of ethereal oils are dissolved all at once or distributed in $C_{2-4}$ alkanol per 100 ml of solution.

3. Process according to claim 1 or 2, characterised in that 0.1 to 1 mg of vitamins and inorganic salts are used as additional components.

4. Process according to claim 1, 2 or 3 characterised in that
the vegetable tannins used are pyrocatechol, tannin, gallic acid, digallic acid and/or pentadigalloyl glucose,
the sugar used is glucose, fructose, ribose, arabinose, rhamnose and/or xylose,
the compounds of the anthocyan type used are anthocyan and/or cyanidine,
the compound of the flavone type used is quercetine,
the ethereal oil used is geraniol 40 to 90%, nerol 4 to 15%, citronellol 10 to 50%, eugenol, linalool or the corresponding aldehydes,
the vitamins used are vitamins A, $B_1$, $B_2$, $B_6$, $B_{12}$, C, K and/or P,
the trace elements used are manganese, magnesium, calcium, iron, zinc, copper, molybdenum, cobalt and/or nickel,
the plant hormones used are auxin and/or kinetin in ng amounts,
the enzymes with an oxidative activity used are peroxidase and/or catalase, and
the inorganic salts used are phosphates and/or chlorides.

5. Process according to claim 1, 2, 3 or 4, characterised in that the solvent used is 70 to 100%, preferably 96%, ethanol.

23